## Europäisches Patentamt

## European Patent Office

## Office européen des brevets

(19)

(11) Veröffentlichungsnummer: **0 094 576**
**B1**

(12) **EUROPÄISCHE PATENTSCHRIFT**

(45) Veröffentlichungstag der Patentschrift:
**01.04.87**

(21) Anmeldenummer: **83104495.3**

(22) Anmeldetag: **06.05.83**

(51) Int. Cl.⁴: **G 01 N 11/04, G 01 N 11/08**

(54) **Vorrichtung zur Bestimmung der Fliessschubspannung von Suspensionen, insbesondere Blut.**

(30) Priorität: **13.05.82 DE 3218037**

(43) Veröffentlichungstag der Anmeldung:
**23.11.83 Patentblatt 83/47**

(45) Bekanntmachung des Hinweises auf die Patenterteilung:
**01.04.87 Patentblatt 87/14**

(84) Benannte Vertragsstaaten:
**AT BE CH DE FR GB IT LI LU NL SE**

(56) Entgegenhaltungen:
**DE - A - 2 622 375**
**FR - A - 2 345 716**
**US - A - 3 908 442**

*Die Akte enthält technische Angaben, die nach dem Eingang der Anmeldung eingereicht wurden und die nicht in dieser Patentschrift enthalten sind.*

(73) Patentinhaber: **Kiesewetter, Holger, Dr.,**
**Altenhoferstrasse 14, D-5524 Usch (DE)**

(72) Erfinder: **Jung, Friedrich, Dipl.-Ing., Gartenstrasse 3,**
**D-5100 Aachen (DE)**
Erfinder: **Radtke, Hartmut, Dr., Schlossstrasse 5,**
**D-5100 Aachen (DE)**
Erfinder: **Witt, Reinhard, Mauerstrasse 92,**
**D-5100 Aachen (DE)**
Erfinder: **Kiesewetter, Holger, Dr., Altenhoferstrasse 4,**
**D-5524 Usch (DE)**

(74) Vertreter: **Görtz, Dr. Fuchs, Dr. Luderschmidt**
**Patentanwälte, Sonnenberger**
**Strasse 100 Postfach 26 26, D-6200 Wiesbaden (DE)**

**Beschreibung**

Die Erfindung betrifft eine Vorrichtung zur Bestimmung der Fliessschubspannung von Suspensionen, insbesondere Blut mit einer ein Netzwerk kapillarer Kanäle aufweisenden Einrichtung.

Blut kann bekanntlich als eigenständiges mobiles Organ verstanden werden, das aus verschiedenen Zellen besteht, die in einer flüssigen Phase, nämlich dem Plasma, suspendiert sind. Die Hauptfunktion des Blutes besteht darin, Sauerstoff und Nährstoffe zu den Parenchymzellen zu transportieren und Kohlendioxid und Stoffwechselmetaboliten abzutransportieren. Daneben hat das Blut noch eine Vielzahl weiterer Aufgaben, zu denen die Wärmeverteilung, die Vehikelfunktion für Hormone, die Abwehr gegen Krankheitserreger und körperfremde Stoffe u.dgl. gehören. Dabei kann man diese Aufgaben unter dem Begriff der Homöostase, der Aufrechterhaltung eines dynamischen (Fliess-)Gleichgewichts, zusammenfassen.

Um eine Homöostase in allen Organen zu gewährleisten, ist ein Mindestfluss des Blutes in den Gefässen notwendig, in denen der Stoffaustausch erfolgt, also in den Mikrozirkulationsgebieten. Der Fluss wird normalerweise in erster Linie durch Änderung des Gefässdurchmessers der Arteriolen geregelt, in geringerem Mass auch durch Variation des treibenden Drucks, durch Regelation der Herzauswurfleistung. Die Effektivität dieser Regulation leitet sich aus dem Hagen-Poiseuilleschen Gesetz ab, gemäss dem die Flussrate von der vierten Potenz des Radius abhängig ist.

Bei Zuständen jedoch, in denen diese Regulationsmöglichkeiten voll ausgeschöpft sind, etwa bei starker Sklerosierung der Gefässe oder hinter Gefässstenosen, bei denen es zur Weitstellung der Arteriolen gekommen ist, tritt ein weiterer Faktor in den Vordergrund, nämlich die innere Reibung oder Viskosität des Blutes. Die Blutviskosität ist für eine geregelte Temperatur keine konstante Grösse, sondern hängt stark von den Scherkräften in den Blutgefässen ab. Bei verlangsamtem Fluss, also kleinen Scherkräften, ist die Viskosität stark erhöht und kann so zum limitierenden Faktor für die Durchblutung werden. Es ist dabei unumstritten, dass dicht gepackte Zellsuspensionen mit Hämatokritwerten über 0,7 bei kleinen Schubbelastungen das Verhalten eines Festkörpers aufweisen können, während dieselben Suspensionen bei hoher Schubspannung (erhöhten Fliessgeschwindigkeiten) eine erstaunlich grosse Fluidität aufweisen.

Umstritten ist jedoch, ob auch normales Vollblut mit einem Hämatokritwert von 0,45 einen Fliesspunkt aufweist, sich also bei kleiner äusserer Belastung wie ein Festkörper und nach Überschreiten einer mechanischen Schwelle durch Erhöhung der Schubspannung wie eine Flüssigkeit verhält.

Dabei liegt ein Fliesspunkt dann vor, wenn bei einem endlichen treibenden Druck das untersuchte Fluid nicht fliesst. Anders ausgedrückt hat das Fluid unterhalb einer Grenzfliessschubspannung $\tau_y$ eine unendlich grosse Viskosität und zeigt somit ein Festkörperverhalten. Zur exakten Definition dieser Fliessschubspannung gehört die genaue Feststellung des Begriffs «Flussstillstand». Bekanntlich hängt der aus makroviskosimetrischen Messungen extrapolierte Fliesspunkt des Blutes von der Fibrinogenkonzentration und somit von der Erythrozytenaggregation, ausserdem jedoch auch sehr stark vom Hämatokritwert und wie erst neuerdings bekannt, von der Erythrozytenverformbarkeit ab.

Nun gewinnt das Phänomen des «Fliesspunktes» von Blut in der Diagnostik und Therapieüberwachung von Patienten mit Fliessanomalien an Interesse, da man jetzt therapeutisch in vivo den Fibrinogengehalt und den Hämatokritwert und damit zwei Determinanten des in vitro messbaren Fliesspunkts verändern kann.

In den vergangenen Jahren ist eine Vielzahl von Methoden zur Messung der Fliessschubspannung entwickelt worden. Dabei ist es üblich, die verschiedenen Bestimmungsmethoden der Fliessschubspannung in statische und dynamische Methoden einzuteilen.

Bei den statischen Verfahren befindet sich das untersuchte Fluid zuerst in Ruhe ($\dot{\gamma}=0$). Die Schubspannung wird anschliessend soweit erhöht, bis das Fluid zu fliessen beginnt.

Bei den dynamischen Verfahren bewegt sich das Fluid zuerst; dann wird die Schubspannung soweit erniedrigt, bis ein Flussstillstand erreicht wird.

Man kann vermuten, dass man mit den statischen Methoden bei konstantem Hämatokritwert höhere Werte für $\tau_y$ erhält als mit den dynamischen. Die Gründe hierfür sind:

a) Die Desaggregation benötigt höhere Schubspannungen als die Aggregation (Überwindung der Haftkräfte oder Haftreibung).

b) Die Aggregate werden in Stase grösser und verfestigen sich.

c) Der funktionelle Hämatokritwert, d.h. die hydrodynamisch effektive Volumenfraktion der Erythrozyten, ist in Stase wegen der grösseren Aggregate höher (Einschluss von Plasma in den Aggregaten, das nicht geschert werden kann).

Vor allem die dynamische Messung wird dadurch kompliziert, dass es zu starken Sedimentationseffekten und damit evtl. sogar zur lokalen Hämokonzentration kommt, was zu einer Erhöhung der Fliessschubspannung führen kann. Allerdings kann in diesem Fall in streng rheologischem Sinne nicht mehr von einer Fliessschubspannung gesprochen werden, da diese nur bei einer unveränderten Zusammensetzung der betrachteten Suspension definiert ist. Wegen der unvermeidlichen Sedimentation von Blut kann man bei Verwendung der dynamischen Methode allenfalls von einer Fliessschubspannung nach erfolgter Hämokonzentration sprechen. Da dieser Sachverhalt bei Durchblutungsstörungen eine grosse Rolle spielen kann, ist es sinnvoll, von der in der allgemeinen Rheologie üblichen strengen Definition des Fliesspunktes abzugehen.

Zur Quantifizierung der Fliessschubspannung wurden in der Vergangenheit Balance-, Kapillar-und Rotationsviskosimeter und eine Sedimentationskammer verwendet. Das Balanceviskosimeter ist beispielsweise in Biorheology 6 (1969) S. 23–32, ein Kapillarviskosimeter in Biorheology 5 (1968) S. 263–270, ein Rotationsviskosimeter in Biorheology 7 (1970) S. 129–135 und eine Sedimentationskammer in NATURE 216 (1967) S. 1121–1123 beschrieben. Nachstehend sind die Vor- und Nachteile dieser Methoden kurz erläutert.

Bei den Balance-Methoden wird über eine Feinwaage ein Tauchkörper höherer Dichte in die zu untersuchende Flüssigkeit gehängt. Auf ihn wirken Gewichtskraft und Auftriebskraft, die im statischen Gleichgewicht sind. Um ihn aus der Ruhelage auszulenken, muss eine Kraft aufgebracht werden, mit deren Hilfe man einen evtl. vorhandenen elastischen Widerstand gegen die Bewegung messen kann. Die maximale Kraft, mit der die Flüssigkeit einer Bewegung des Tauchkörpers entgegenwirkt, ist die Fliessschubkraft $F_y$. Umgekehrt ist im dynamischen System der Tauchkörper zuerst in Bewegung. Nach dem Stopp der Bewegung lässt sich die Fliessschubkraft mit Hilfe der Gleichgewichtsbedingung ermitteln, wobei die Fliessschubspannung bei bekannter Oberfläche des Körpers A errechnet werden kann. Diese Methode weist jedoch bei der Anwendung auf Blut extrem schwankende Resultate auf. So schwanken sorgfältig aufgenommene und vollständig wiedergegebene Resultate im Mittel um 40%, zum Teil jedoch um mehr als 400%.

Bei der statischen Sedimentationsmethode wird eine nach oben konisch zulaufende Glasröhre verwendet, deren Innendurchmesser zwischen 2 und 0,08 mm beträgt. Die Glasröhre wird mit Blut gefüllt und etwa 10 Minuten vertikal aufgestellt. Unterhalb eines bestimmten Röhrendurchmessers $d_y$ sedimentiert eine Säule von Erythrozytenaggregaten ab; oberhalb dieses Durchmessers bleibt die Erythrozytensäule in Ruhe. Für diesen Durchmesser wird unter Vernachlässigung des Kegelwinkels eine Kräftebilanz erstellt, aus der sich dann die Fliessschubspannung ermitteln lässt. Bei dieser Methode wird jedoch nur die Schubspannung ermittelt, die zum Abriss von Aggregaten innerhalb einer sedimentierten Erythrozytensäule führt, so dass man mit dieser Methode nicht den Fliesspunkt des Blutes, sondern nur den der Aggregate ermitteln kann.

Eine weitere, bereits vorstehend erwähnte Methode ist die Fliesspunktbestimmung mit Rotationsviskosimetern. Diese von den technischen Wissenschaften zur Verfügung gestellten Rotationsviskosimeter mit unterschiedlicher Geometrie finden seit langem auch für hämorheologische Untersuchungen Verwendung. Den bisher üblichen Geräten ist gemeinsam, dass verschiedene, meist konstante Rotationsgeschwindigkeiten und damit endliche Schergrade ($\dot{\gamma}$–$10^{-3}$–$10^2$ 1/s) vorgegeben werden. Durch das Fluid werden Drehmomente und damit Schubspannungen auf einen Messfühler übertragen.

Nach der in die hämorheologische Literatur eingeführten Casson-Gleichung, mit der das Fliessverhalten über einen weiten Schergradbereich durch die Beziehung

$$\sqrt{\tau} = k_1 + k_2\sqrt{\dot{\gamma}}$$

beschreibbar ist, wurde verschiedentlich die Fliesskurve zu $\dot{\gamma} = 0$ extrapoliert, wobei $\tau_y = k_1^2$ (bei Schergraden von $10^{-2}$ bis $50\,\mathrm{s}^{-1}$) errechnet wurde. Dieses Verfahren ist häufig kritisiert worden, wird jedoch vielfach in der Literatur dennoch verwendet.

Die prinzipielle Schwierigkeit der bisherigen Fliessschubspannungsmessungen besteht darin, dass Rotationsviskosimeter verwendet werden, bei denen ein endlicher Schergrad, also gleichsam ein Fluss vorgegeben wird, ein eventuell vorhandener Fliesspunkt also messtechnisch nicht erfasst werden kann, sondern nach verschiedenen Arten extrapoliert werden muss.

Eine Verbesserung der Messmethode mit Rotationsviskosimetern stellt ein Viskosimeter von DEER dar, bei dem im Gegensatz zu den meisten anderen bisher verwendeten Rotationsviskosimeters das Drehmoment und somit die Schubspannung (gleichsam der Druck) vorgegeben ist und somit der Schergrad bestimmt ist. Dieser kann auch Null betragen, so dass die Fliessschubspannung $\tau_y$ in einem makroskopischen Bereich direkt gemessen werden kann. Eine derartige Messung kann somit nur Aussagen für diesen Bereich machen und allenfalls zur Extrapolation für den Mikrozirkulationsbereich herangezogen werden.

Eine weitere Möglichkeit zur Bestimmung der Fliessschubspannung ist die Untersuchung der Abhängigkeit der Geschwindigkeit v vom Druckgradienten $\Delta p$ in Kapillarviskosimetern. Es lässt sich eine Extrapolation dieses $\Delta p$-v-Diagramms bzw. nach Umrechnung der Daten das $\dot{\gamma}$-$\tau$-Diagramm für v = 0 bzw. $\dot{\gamma} = 0$ durchführen. Verwendet werden dabei Kapillaren mit einem Durchmesser von 0,05 bis 1 mm und einer Länge bis zu 8 m. Hauptnachteil dieser Methode ist, dass die meisten Messungen ohne optische Beobachtung des Erythrozytenflusses gemacht wurden. Wie die Beobachtungen von DEVENDRAN, PROC. ASME 73 – WA/BIO 35 (1973), S. 1–4, zeigen, ist bei den Kapillarviskosimetern kein Fliesspunkt bestimmbar, da es nie zu einem Flussstillstand kommt.

In der US-A-3 908 442 ist eine Vorrichtung zur Bestimmung der Viskosität, insbesondere der Druckdifferenz von fliessfähigen Substanzen in unterschiedlich langen Röhren beschrieben. Dabei werden die Substanzen durch die Röhren gedrückt, wobei die sich einstellende Druckdifferenz zwischen beiden Röhren gemessen wird.

Somit handelt es sich also um ein dynamisches Verfahren mit gleichem Pumpvorschub, d.h. mit dieser Vorrichtung kann nicht die Fliessschubspannung einer Suspension, insbesondere nicht von Blut, gemessen werden.

Nach wie vor ist daher die Frage nicht gelöst, ob menschliches Blut eine endliche Fliessschubspannung hat.

Der Erfindung liegt daher die Aufgabe zugrunde, eine Vorrichtung der eingangs erwähnten Art zur Verfügung zu stellen, mit der die Fliessschubspannung von Suspensionen, insbesondere Blut, in Bereichen simuliert werden kann, die ein Netzwerk kapillarer Kanäle aufweisen.

Diese Aufgabe wird durch die Merkmale des Anspruchs 1 gelöst.

Erfindungsgemäss wird nunmehr eine Messapparatur zur Verfügung gestellt, mit der es auf einfache und reproduzierbare Weise möglich ist, die Fliessschubspannung von Blut gemäss der statischen oder dynamischen Methode zu bestimmen und bei Bedarf die Fliesskurve des Blutes, insbesondere im Bereich kleiner Schubspannungen ($\tau < 500\,mPa$), aufzuzeichnen.

Durch die Auswertung der Fliesskurve ist es möglich, das nicht-Newtonsche Verhalten von Blut zu beobachten und quantitativ zu erfassen. So ist beispielsweise das Verhalten von Blut nicht nur von der Temperatur, sondern auch von den einwirkenden Scherkräften abhängig. Die Gründe hierfür sind das Verhalten der suspendierten bzw. emulgierten Teilchen, nämlich deren Deformierung und Orientierung, und ihre Wechselwirkungen miteinander, so vor allen Dingen die Aggregation. Bei idealen Newtonschen Fluiden steigt die Scherrate linear mit steigender Schubspannung an, d.h. sie haben eine konstante Viskosität, die als Quotient von Schubspannung und Scherrate gebildet ist. Trägt man dagegen bei Vollblut die Schubspannung über der Scherspannung auf, so zeigt sich, dass die Steigung der Kurve nicht konstant ist. Diese Steigung wird als «scheinbare» («apparente») Viskosität $\eta_{app}$ bezeichnet.

Nun zeigt das Blut bei kleinen Schubspannungen das Verhalten der sogen. Dilatanz, d.h. die apparente Viskosität nimmt bei steigender Schubspannung zu, und bei höheren Schubspannungen ein strukturviskoses Verhalten an. Mit dem erfindungsgemässen Erythrozyten-Stase-Messgerät (ESM) kann die Dilatanz von Blut – eine Grösse, die auch rheologische Bedeutung hat – aus der für kleine Schubspannungen ermittelten Fliesskurve bestimmt und somit quantitativ erfasst werden.

Eine weitere Eigenschaft des Blutes, nämlich die zeitliche Zunahme der apparenten Viskosität (Rheopexie) kann mit dem erfindungsgemässen Viskosimeter einfach optisch beobachtet und gegebenenfalls quantitativ erfasst werden.

Zu den wesentlichen Elementen des erfindungsgemässen ESM gehört die Messeinrichtung, die vorzugsweise in Form einer Messkammer vorliegt. In dieser Messkammer wird der natürliche Mikrozirkulationsbereich durch ein künstliches in vitro-Netzwerk, das vereinfacht vorliegt, simuliert. Dieses künstliche Netzwerk besteht im wesentlichen aus wenigstens zwei Kanälen, die vorteilhafterweise von einem Zuführungskanal in einem beliebigen Winkel abzweigen. Wesentlich an dieser Anordnung ist, dass die beiden kapillaren Kanäle eine unterschiedliche Länge aufweisen. Die unterschiedliche Länge der Kanäle soll dem invivo-Verhalten von Blut in Mikrozirkulationsgebieten mit unterschiedlichem hydrodynamischen Widerstand Rechnung tragen. Bekanntlich existiert das Stasis-Phänomen, bei dem Blut zwar in relativen kurzen, nicht jedoch in langen vorzugsweise dazu parallelgeschalten kapillaren Kanälen fliesst.

Mit der vorliegenden Messkammer kann dieses Verhalten von Blut in Mikrozirkulationsbereichen erfolgreich simuliert werden. So kann mit der erfindungsgemässen Vorrichtung festgestellt werden, dass Blut mit einer bestimmten Zusammensetzung noch in kurzen Kanälen bei bestimmten Schubspannungen fliesst, jedoch in langen Kanälen noch im Zustand des Stillstands vorliegt. Die Messgrösse, bei der auch das Blut in den langen Kanälen zu fliessen beginnt, ist ein Kriterium für die Fliessfähigkeit des Blutes in der minderperfundierten Mikrozirkulation.

Die Erfindung wird, anhand der nachfolgenden Beschreibung, von Ausführungsformen unter Bezugnahme auf die Zeichnung näher erläutert.

Es zeigen

Fig. 1 eine perspektivische Ansicht der erfindungsgemässen Vorrichtung;

Fig. 2 eine vergrösserte Schnittansicht entlang der Linie II–II durch die Vorrichtung gemäss Fig. 1, wobei jedoch diese zugeklappt ist;

Fig. 3 eine Draufsicht auf eine Messkammer, die in der Vorrichtung gemäss Fig. 1 und 2 vorgesehen ist und

Fig. 4 einen Längsschnitt durch eine weitere Ausführungsform einer Messkammer.

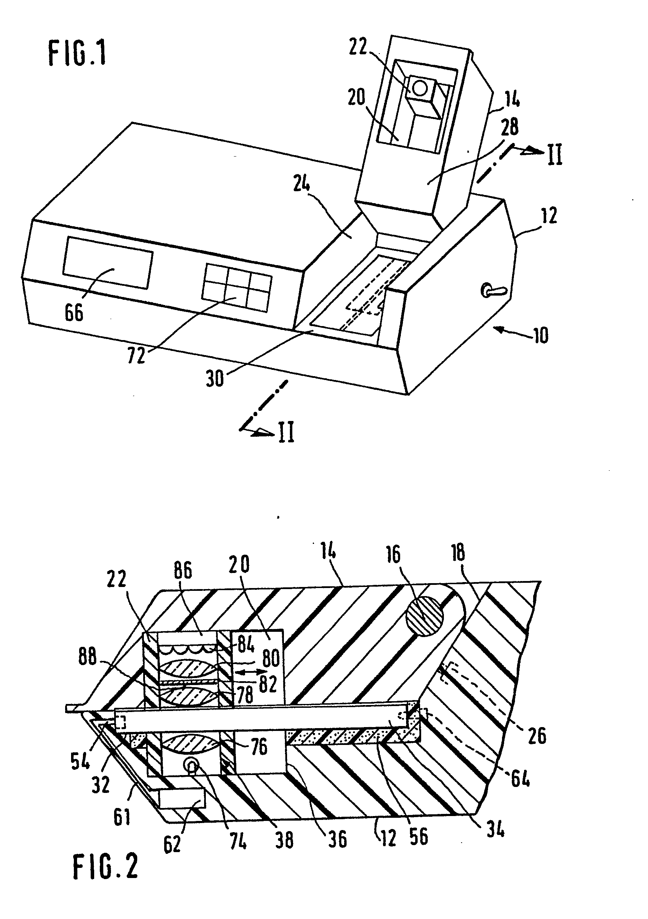

In Fig. 1 ist die erfindungsgemässe Vorrichtung mit 10 bezeichnet. Diese Vorrichtung 10 besteht im wesentlichen aus einem Gehäuse 12, das einen aufklappbaren Deckel 14 aufweist. Dieser Deckel 14 ist um die Horizontalachse 16 klappbar und so ausgestaltet, dass er sich im aufgeklappten Zustand an die im Gehäuse 12 vorgesehene Schräge 18 anlehnen und dort abstützen kann.

Der Deckel 14 weist auf seiner Unterseite eine Ausnehmung 20 auf, in der eine Detektoreinheit 22 vorgesehen ist, die gemäss einer bevorzugten Ausführungsform horizontal verschiebbar ist.

Der Deckel 14 selbst schliesst bündig mit einer im Gehäuse 12 vorgesehenen Öffnung 24 ab und betätigt im geschlossenen Zustand einen an der Schräge 18 vorgesehenen elektrischen Kontakt 26, der die Vorrichtung 10 betriebsbereit macht.

Die Öffnung 24 weist eine für die Unterseite 28 des Deckels 14 dienende horizontale Auflagefläche 30 auf, die eine im wesentlichen rechteckige Aussparung 32 zur Aufnahme der Messkammer 34 aufweist. Dabei nimmt die Messkammer 34, wie aus Fig. 1 ersichtlich, im wesentlichen die gesamte Aussparung 32 ein und bildet zusammen mit der Auflagefläche 30 eine einheitliche Oberfläche.

Unterhalb der Messkammer 34 und sich an die Aussparung 32 anschliessend ist eine weitere Ausnehmung 36 vorgesehen, innerhalb der eine weitere Detektoreinheit 38 angeordnet ist, die mit der Detektoreinheit 22 entweder fest verbunden ist oder aber mit dieser synchronisiert ist. Dabei ist diese Detektoreinheit 38 ebenso wie die Detektoreinheit 22 innerhalb der Ausnehmungen 36 bzw. 20, die im wesentlichen vertikal fluchten, horizontal verschiebbar, wie durch den Pfeil in Fig. 2 gezeigt, und befinden sich jeweils in unmittelbarer Nachbarschaft zur Oberseite 40 und zur Unterseite 42 der Messkammer 34.

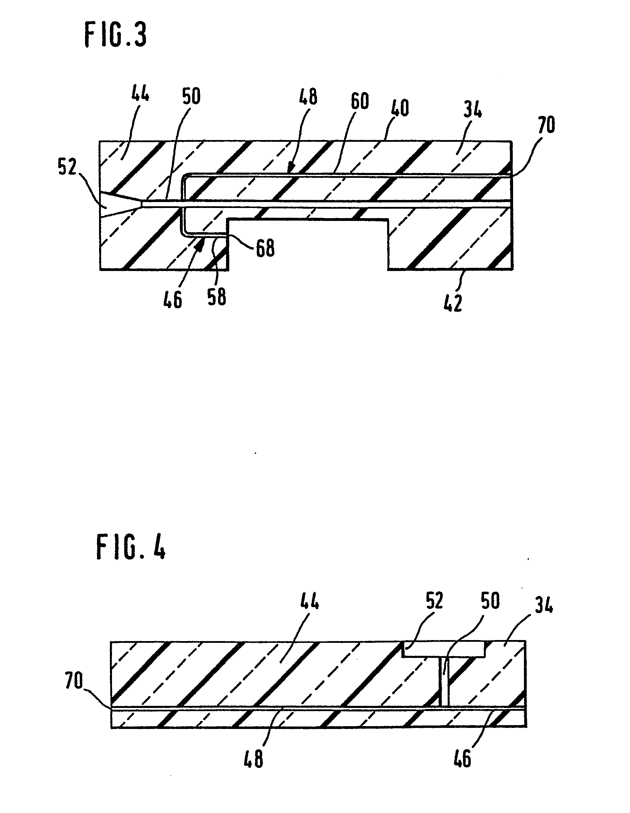

Gemäss den in Fig. 3 und 4 gezeigten Ausführungsformen besteht die Messkammer 34 im wesentlichen aus dem Festkörper 44, in den vorzugsweise zwei Kanäle 46 und 48 eingearbeitet sind. Diese Kanäle 46 und 48 sind im wesentlichen kapillar ausgeführt, d.h. sie besitzen einen Durchmesser von etwa 15–1000, insbesondere 30–150 µm.

Die Form des Querschnitts dieser Kanäle 46 und 48 ist im wesentlichen unkritisch und soll vorzugsweise oval bis kreisrund sein.

Das Verhältnis der Länge $L_l$ des langen Kanals 48 und der Länge $L_k$ des kurzen Kanals 46 liegt in einem Bereich von 3 : 1–15 : 1, vorzugsweise 6 : 1–10 : 1, insbesondere etwa 7 : 1.

In den in Fig. 3 und 4 gezeigten Ausführungsformen zweigen die Kanäle 46 und 48 von einem Hauptkanal 50 ab, über den die zu untersuchende Flüssigkeit, insbesondere Blut, zugeführt wird. Dieser Kanal 50 weist eine Zuführungsöffnung 52 auf, die gegenüber dem Querschnitt des Hauptkanals 50 erweitert ist. Diese Zuführungsöffnung 52 kann entweder die in Fig. 3 gezeigte konische Form besitzen oder aber die in Fig. 4 gezeigte zylindrische Form aufweisen, wobei letztere mit dem Hauptkanal 50 konzentrisch ist.

Diese Zuführungsöffnung 52 steht mit einer im Gehäuse 12 vorgesehenen Zuführungseinrichtung 54 in Verbindung, durch die aus einem nicht gezeigten Reservoir die zu untersuchende Flüssigkeit, insbesondere Blut, der Messkammer 34 zugeführt wird.

Gemäss der in Fig. 2 gezeigten Ausführungsform wird die Zuführungsöffnung 52 beim Einlegen der Messkammer 34 zunächst mit der Zuführungseinrichtung 54 in Verbindung gebracht. Anschliessend wird das der Zuführungsöffnung gegenüberliegende Ende der Messkammer 34 in die Aussparung 32 eingelegt, wobei das in der Aussparung 32 vorgesehene elastische Element 56, das vorzugsweise aus einem geschäumten Kunststoff besteht, einen druckdichten Sitz zwischen der Zuführungseinrichtung 54 und der Zuführungsöffnung 52 sicherstellt.

Der Durchmesser des Hauptkanals 50 ist vorteilhafterweise grösser als der Durchmesser der Kanäle 46 und 48 und beträgt dabei 30–2000, insbesondere 60–300 µm. Seine Form entspricht dabei der Form der vorstehend erläuterten Form der Kanäle 46 und 48.

Die Kanäle 46 und 48 können jeweils mit einem unterschiedlichen Winkel vom Hauptkanal 50 abzweigen. Vorzugsweise zweigen sie etwa im rechten Winkel vom Hauptkanal 50, wie in Fig. 3 und 4 gezeigt, ab.

Gemäss einer ersten Ausführungsform, die in Fig. 3 gezeigt ist, zweigen die Kanäle 46 und 48 jeweils nochmals unter einem rechten Winkel ab und bilden dabei in etwa eine U-Form. Dabei verlaufen die abgezweigten Äste 58 und 60 in etwa parallel.

In einer zweiten Ausführungsform, die in Fig. 4 gezeigt ist, existiert eine derartige zweite Abzweigung nicht, so dass die Kanäle 46 und 48 im wesentlichen eine gemeinsame Längsachse besitzen.

Die Herstellung einer Verzweigung zwischen dem Hauptkanal 50 einerseits und den Seitenkanälen 46 und 48 andererseits stellte bisher ein ungelöstes Problem dar und wird gemäss dem nachfolgend geschilderten Verfahren gelöst.

Die Herstellung des Netzwerkes erfolgt im wesentlichen durch Giessen mit einem giessfähigen, im wesentlichen lichtdurchlässigen Kunststoff, beispielsweise mit Polyester. Ein derartiger giessfähiger, blutverträglicher Kunststoff wird unter dem üblichen Zusatz von Beschleunigungs- und Härtungsmitteln eingesetzt und darf beim Aushärten nur geringfügig schrumpfen. Desweiteren muss gewährleistet sein, dass beim Mischen und Giessen entstandene Lufteinschlüsse in Form von Blasen vor dem Härten an die Oberfläche gelangen und somit entfernt werden können.

Ein derartiger Kunststoff wird in eine entsprechend vorbereitete Form gegossen, die im wesentlichen der der Messkammer 34 entspricht.

Kapillare Kanäle werden in den Festkörper 44 dadurch eingearbeitet, dass man ungereckte Fäden, deren Querschnitt und Durchmesser in etwa den geforderten Dimensionen der Kanäle 46 und 48 entsprechen, in der Form mit der gewünschten Kanalführung anordnet und anschliessend die giessfähige Masse einfüllt. Als Material für derartige Fäden kann beispielsweise Polyamid eingesetzt werden. Vorzugsweise setzt man monofile Nylonfäden ein, die vorteilhafterweise mit einem entsprechenden Trennmittel, beispielsweise PTFE, versehen sind.

Um eine entsprechende Verzweigung mit dem als Hauptkanal 50 dienenden Hauptfaden herzustellen, wird dieser mit dem zur Herstellung der Kanäle 46 und 48 eingesetzten Faden durchbohrt, was vorteilhafterweise unter dem Mikroskop mit einer entsprechend dimensionierten Nadel geschieht, in die der zur Herstellung der Kanäle 46 und 48 dienende Faden eingelegt ist. Eine derart hergestellte Verzweigung wird entsprechend der vorstehenden Erläuterung in der Gussform derart fixiert, dass sich die gewünschte Geometrie ($L_l/L_k$) ergibt. Diese Fäden werden aus der Form herausgeführt und stehen somit aus dem ausgehärteten Kunststoffblock, der den Festkörper 44 bildet, hervor.

Da ungereckte Fäden eingesetzt werden, können diese durch Recken, also durch Ziehen, aus dem ausgehärteten Kunststoffblock entfernt werden, wobei die kapillaren Kanäle 46, 48 und 50 zurückbleiben.

Mit dieser Herstellungsweise ist es erstmals möglich, in-vitro-Netzwerke mit echten Verzweigungen herzustellen, in denen der Mikrozirkulationsbereich, in dem das Blut geführt wird, simuliert werden kann.

Neben den in Fig. 3 und 4 gezeigten Anordnungen der Kanäle 46, 48 und 50 können natürlich beliebig komplizierte Geometrien im Prinzip hergestellt werden.

So ist es in einer speziellen Ausführungsform durchaus denkbar, dass der kurze Kanal und der lange Kanal jeweils eine gemeinsame Eintritts- und Austrittsöffnung besitzen. Dabei ist der kurze Kanal im wesentlichen gestreckt, während der lange Kanal in beliebigen Windungen oder geometrischen Anordnungen innerhalb des Festkörpers 44 geführt ist. Aus Gründen der Messtechnik ist es jedoch bevorzugt, dass derartige Windungen im wesentlichen in der Horizontalebene bzw. der Messebene liegen.

Gemäss dieser Ausführungsform können die Eintritts- und Austrittsöffnungen jeweils eine Zuführungsöffnung aufweisen, die im wesentlichen der Zuführungsöffnung 54 entspricht. Es kann natürlich auch ebenfalls eine Austrittsöffnung mit derselben Form vorgesehen sein. Da in dieser Ausführung ein Hauptkanal mit einer entsprechenden Verzweigung nicht vorgesehen ist, kann die Zuführungsöffnung zusätzlich in einen kurzen Hauptkanal übergehen, der direkt in den kurzen und langen Seitenkanal übergeht.

Anstelle der vorstehend beschriebenen Fäden zur Herstellung der Kanäle 46, 48 und 50, können bereits industrieseitig gefertigte Hohlfäden eingesetzt werden, die beispielsweise bei der Dialyse zum Einsatz kommen. Derartige Hohlfäden besitzen die vorstehend erwähnten Dimensionen für den Durchmesser und die Form der Kanäle. Sofern hydrophile Hohlfasern, beispielsweise aus Zellulosederivaten, eingesetzt werden, ist natürlich ein derartiger Faden vollständig in eine Kunststoffmatrix einzugiessen, um ein Ausdringen von Plasma aus den Mikroporen zu verhindern. Ein derartiges Ausdringen kann mit Hohlfäden aus einem hydrophoben Material, beispielsweise Polypropylen, verhindert werden, so dass sich ein vollständiges Ausgiessen des Festkörpers 44 erübrigen kann. Es hat sich jedoch als vorteilhaft erwiesen, dass die Kanäle 46 und 48 in einer Matrix, beispielsweise der Kunststoffmatrix, fixiert werden, um die mit optischen Hilfsmitteln durchgeführte Messung möglichst unproblematisch zu gestalten.

Neben der Herstellung von Fadenkreuzungen mit Hilfe einer Nadel können natürlich weitere Methoden, wie Bohren, Stechen oder Elektronstrahltechnik, zur Anwendung kommen.

Da jedoch an die Porengeometrie und an die Reproduzierbarkeit der Porendurchmesser besondere Anforderungen gestellt werden müssen, bietet sich besonders die Kernspurtechnik an. Bei diesem Verfahren wird an der Stelle des gewünschten Loches ein einzelnes, energiereiches Schwerion hindurchgeschossen und die sich entlang der Teilchenbahn gebildete «Radikal»-Spur in einem zweiten Herstellungsprozess auf den gewünschten Durchmesser aufgeätzt.

Durch entsprechende Dimensionierung eines solchen Teilchens kann erreicht werden, dass keine durchgehende Bohrung, sondern lediglich ein Sackloch erzeugt wird, wie dies beispielsweise in Fig. 4 gezeigt ist.

Die Messkammer 34 ist vorzugsweise als sogen. Einmalteil bzw. Wegwerfteil ausgeführt, d.h. sie wird nach Gebrauch aus der Vorrichtung 10 entnommen und weggeworfen. Es muss nicht hinzugefügt werden, dass das nichtgezeigte Blutreservoir natürlich auch in die Messkammer 34 integriert sein kann, also beispielsweise unmittelbar vor der Zuführungsöffnung 52 und der Zuführungseinrichtung 54 angeordnet sein kann.

Die Zuführungseinrichtung 54 ist über eine Leitung 61 mit dem Druckgenerator 62 in Verbindung, der den für die Befüllung der Messkammer 34 und für die Durchführung der Messung notwendigen Druck erzeugt. So wird zunächst die Suspension in das Netzwerk mit einem hohen Druck von etwa 1 mH$_2$O = 10$^4$ Pa eingefüllt. Anschliessend kann dieser Druckgenerator 62 den Druck auf 0 senken, wobei eine Drucksteigerung in Stufen von etwa 2,5 Pa und darüber erfolgen kann.

Sofern die in Fig. 3 gezeigte Ausführungsform mit Blut gefüllt wird, muss natürlich der Hauptkanal 50 nach dem Füllen geschlossen werden, was durch den Stopfen 64 erfolgen kann, der in der Messkammer 34 vorgesehen werden kann. Dieser Stopfen kann beispielsweise als elektromagnetischer Verschluss vorliegen.

Für die Messung werden maximal 2 ml venöses Vollblut mit einem Hämatokritgehalt > 0,4 im antikoagulierten Zustand benötigt. Dieses Blut wird vor der Messung in die Messkammer insgesamt eingefüllt, also über den Hauptkanal 50 in die Seitenkanäle 46 und 48. Das Befüllen kann entweder ausserhalb der Vorrichtung oder innerhalb der Vorrichtung geschehen. Vorzugsweise wird man zur Vereinfachung die Messkammer 34 ausserhalb der Vorrichtung füllen und anschliessend in die Aussparung 32 einlegen. Wie bereits vorstehend erläutert, wird der Messvorgang mit dem Schliessen des Deckels 14 gestartet und läuft im wesentlichen automatisch ab. Dabei wird das erhaltene Messergebnis über das Display 66 angezeigt, das im Gehäuse 12 angeordnet ist.

Vor Beginn der eigentlichen Messung erfolgt ein Druckabgleich zwischen dem kurzen und dem langen Kanal 46 und 48 derart, dass die Druckdifferenz, die über den Kanälen anliegt, auf Null abgeglichen wird. Dies geschieht durch Variation des aufgegebenen Drucks. Dabei wird der Druck als Null definiert, der für eine bestimmte Mindestzeit, beispielsweise eine Minute, keinen Fluss im kurzen Kanal bewirkt. Nach diesem Nullabgleich kann die Bestimmung der Fliessschubspannung beginnen. Zu diesem Zweck muss der Druck ermittelt werden, bei dem die Erythrozyten im langen Kanal 48 zu fliessen beginnen bzw. gerade noch nicht fliessen. Zur Ermittlung dieser sogen. statischen Grenzdruckdifferenz $\Delta P_{ys}$ wird der Druck in diskreten Schritten, beispielsweise 1–10, insbesondere 2,5 Pa, erhöht, bis im langen Kanal ein Fluss beobachtet werden kann. Dabei liegt zwischen jeder Druckerhöhung eine Einstellzeit von 0,1–5, insbesondere etwa 1 Minute. Als Fluss wird jede Zellverschiebung im langen Kanal 48 bezeichnet, die schneller ist als 0,1–10, insbesondere 0,5 $\mu$m · min$^{-1}$.

Der aufgebrachte Druck kann dabei im Druckgenerator 62 sowohl pneumatisch als auch vorzugsweise hydrodynamisch aufgebracht werden. Zu diesem Zweck kann in der Messkammer auch vorteilhafterweise ein Flüssigkeitsreservoir vorgesehen sein, das der Druckaufgabe dient. Weiterhin kann der Druckgenerator auch an den Auslassöffnungen 68 bzw. 70 des kurzen bzw. langen Kanals 46 bzw. 48 angeordnet sein, wobei natürlich ein entsprechend dimensionierter Unterdruck mit den vorstehend erwähnten Druckschritten in umgekehrter Richtung angelegt wird.

Sobald eine sichtbare Zellverschiebung im langen Kanal 48 von der nachstehend näher erläuterten Detektoreinheit 22 und 38 detektiert wird, ist der Fliesspunkt überschritten und der Versuch wird automatisch ausgewertet. Zu diesem Zweck wird aus der letzten Druckdifferenz, die noch keinen Fluss bewirkte, die Fliessschubspannung $\tau_y$ als rheologische Grösse berechnet.

Da dieser Wert jedoch stark vom Hämatokritwert der Messprobe abhängt, sollte er zu Vergleichszwecken auf einen Referenzhämatokritwert von beispielsweise 0,45 korrigiert werden. Dieser Hämatokritwert kann entweder in die Vorrichtung 10 über ein Tastenfeld 72 eingegeben werden oder aber vorzugsweise bereits selbst parallel im Gerät zur Messung bestimmt werden, wobei das Tastenfeld entfällt. Mit Hilfe des tatsächlich erhaltenen Hämatokritwertes wird dann eine korrigierte Fliessschubspannung $\tau_{ykorr\,45}$ berechnet und auf dem Display 66 angezeigt.

Da die für Nullpunkteinstellung sowie die Fliesspunktmessung erforderliche Geschwindigkeitsmessung wegen der extrem kleinen Geschwindigkeiten (0,5 $\mu m \cdot min^{-1}$) mit den herkömmlichen Messverfahren nicht realisiert werden konnte, wurde ein Detektorsystem 22, 38 entwickelt, das auch bei kleinsten Geschwindigkeiten eine Entscheidung über Fluss oder Stillstand erlaubt.

Das hierbei zum Einsatz kommende Messverfahren nutzt dabei den Effekt aus, dass sich die Intensität des transmittierten Lichts, das eine Kapillare durchstrahlt, während der Bewegung bzw. beim Übergang von der Stase in den Fluss und umgekehrt wenigstens lokal ändert. Dabei weist die Detektoreinheit 38 eine Lichtquelle 74 auf, an die sich eine aus drei Linsen 76, 78 und 80 bestehende Vergrösserungsoptik anschliesst, die in den Detektoreinheiten 22 und 38 angeordnet sind. Zwischen der Linse 78 und 80 ist eine Vorblende 82 vorgesehen, die einen lokalen Ausschnitt definierter Grösse und Form aus dem Licht begrenzt. Dabei kann die Vorblende sowohl eine kreisrunde als auch rechteckige Öffnung besitzen. Der Durchmesser der kreisrunden Öffnung liegt in einem Bereich von 5–50, insbesondere 10–30 µm, während bei einem Einsatz einer rechteckigen Öffnung die Breite des Spalts 5–50, insbesondere 10–30 µm, und dessen Länge 5–1000, insbesondere 10–150 µm, betragen. Das die Blende 82 passierende Licht wird mit der Linse 80 aufgeweitet und auf den Lichtdetektor 84 projiziert. Dieser kann aus einem einzigen Lichtsensor oder aber vorzugsweise aus einer nxm – Matrix von Lichtsensoren, beispielsweise Halbleitersensoren bestehen. Dem Lichtdetektor ist eine elektronische Verstärkereinheit 86 nachgeschaltet, die kleinste Änderungen des transmittierten Lichts detektiert und der nichtgezeigten elektronischen Auswerteinheit den Zustand der Stase oder des Flusses meldet. Da die Änderung des transmittierten Lichts an Punkten örtlicher Transmissionsextrema am grössten ist, kann die gesamte optische Auswerteinheit zur Erhöhung ihrer Empfindlichkeit gegebenenfalls solange manuell oder mit Hilfe eines Schrittmotors verfahren werden, bis ein solcher Extremwert gefunden ist. Für den kurzen und den langen Kanal 46 bzw. 48 kann je eine eigene optische Auswerteinheit vorgesehen sein. Ist die gesamte Einheit jedoch verfahrbar, so genügt eine einzige Einheit für beide Kanäle. Die dann erforderliche Positionierung erfolgt über die eingebaute Elektronik. Anstelle einer beweglichen Elektronik kann natürlich auch eine Mehrzahl von festen Messpunkten auf der Detektoreinheit 22 vorgesehen sein. Hierzu muss nur die Blende 82 eine Mehrzahl von Öffnungen 88 besitzen, die jeweils von einem Lichtstrahl durchstrahlt werden müssen, der den kurzen bzw. langen Kanal 46 bzw. 48 passiert hat. Derartigen Öffnungen 88 ist dann jeweils ein Lichtdetektor 84 zugeordnet, der jeweils separat mit der Auswerteinheit verbunden ist.

Mit der vorgestellten Geometrie ist es ebenfalls möglich, unter Variation des Kreuzungswinkels und der Durchmesser der Seitenkanäle 46 und 48 eine für die Blutrheologie wichtige und bisher nicht quantifizierbare Grösse, nämlich die Hämatokritverteilung in parallel geschalteten Gefässen, zu quantifizieren. So können beispielsweise in den Kanälen 46 und 48 Elektroden zur Messung der Leitfähigkeit der in den Kanälen 46 und 48 befindlichen Blutsäule vorgesehen sein. Aus dem erhaltenen Leitfähigkeitswert kann dann auf den Hämatokritwert geschlossen werden. Eine derartige Messanordnung ist beispielsweise in der DE-A-3 202 067, veröffentlicht am 04.08.83, beschrieben.

**Patentansprüche**

1. Vorrichtung zur Bestimmung der Fliessschubspannung von Suspensionen, insbesondere Blut, mit einer ein Netzwerk kapillarer Kanäle aufweisenden Einrichtung, gekennzeichnet durch wenigstens zwei kapillare Kanäle (46, 48), bei denen das Verhältnis der Länge $L_1$ des langen Kanals (48) und der Länge $L_k$ des kurzen Kanals (46) in einem Bereich von 3 : 1 bis 15 : 1 liegt, und die über einen Hauptkanal (50) mit einer Einrichtung zur Erzeugung eines variablen Druckgefälles verbunden sind, wobei die Kanäle (46, 48, 50) in einem Festkörper (44) aus einem gegossenen, im wesentlichen lichtdurchlässigen Kunststoff vorgesehen sind, und durch ein verschiebbares optisches Detektorsystem (22, 38), mit dem das Fliessen der Suspension in den kapillaren Kanälen (46, 48) feststellbar ist.

2. Vorrichtung nach Anspruch 1, dadurch gekennzeichnet, dass das Verhältnis etwa 7 : 1 beträgt.

3. Vorrichtung nach Anspruch 1, dadurch gekennzeichnet, dass der Innendurchmesser der Kanäle (46, 48) etwa 15–1000 µm beträgt.

4. Vorrichtung nach Anspruch 1, dadurch gekennzeichnet, dass die Kanäle (46, 48) gewinkelt mit dem Hauptkanal (50) verbunden sind.

5. Vorrichtung nach Anspruch 1, dadurch gekennzeichnet, dass Hohlfäden aus einem hydrophoben Material in dem Festkörper (44) vorgesehen sind, die die Kanäle (46, 48, 50) aufweisen.

6. Vorrichtung nach Anspruch 1, dadurch gekennzeichnet, dass die Einrichtung zur Erzeugung eines variablen Druckgefälles in den Kanälen (46, 48) ein pneumatischer oder hydrodynamischer Druckgenerator (62) ist.

7. Vorrichtung nach Anspruch 1, dadurch gekennzeichnet, dass der Druckgenerator (62) in diskreten Schritten von 1–10 Pa betreibbar ist.

8. Vorrichtung nach Anspruch 1, dadurch gekennzeichnet, dass eine die Kanäle (46, 48) und den Hauptkanal (50) aufweisende Messkammer (34) zwischen dem Detektorsystem (22, 38) vorgesehen ist.

9. Vorrichtung nach Anspruch 8, dadurch gekennzeichnet, dass das Detektorsystem (22, 38) eine Lichtquelle (74), eine Vergrösserungsoptik (76, 78, 80), eine Vorblende (82) und wenigstens einen Lichtdetektor (84) aufweist.

10. Vorrichtung nach Anspruch 9, dadurch gekennzeichnet, dass das Detektorsystem (22, 38) mehreren festen Messpunkten entsprechende Lichtsensoren aufweist.

11. Vorrichtung nach Anspruch 9, dadurch gekennzeichnet, dass das Detektorsystem (22, 38) manuell oder mittels eines Schrittmotors oberhalb und unterhalb der Messkammer (34) verschiebbar ist.

12. Vorrichtung nach Anspruch 9, dadurch gekennzeichnet, dass das Detektorsystem (22, 38) mit wenigstens einer automatischen Auswertungseinheit verbunden ist.

13. Vorrichtung nach einem der Ansprüche 1–12, dadurch gekennzeichnet, dass in wenigstens einem der Kanäle (46, 48) Elektroden zur Bestimmung der Leitfähigkeit der in dem betreffenden Kanal befindlichen Suspensionen angeordnet sind.

14. Verfahren zur Herstellung der ein kapillares Netzwerk aufweisenden Einrichtung für die Verwendung in der Vorrichtung gemäss Anspruch 1, dadurch gekennzeichnet, dass man einen oder mehrere langgestreckte Verdrängungskörper, deren Durchmesser und Form dem Durchmesser und der Form der Kanäle (46, 48) und des Hauptkanals (50) der Einrichtung entsprechen, in einer Form verbunden anordnet, die Form mit einem giess- und aushärtbarem, im wesentlichen lichtdurchlässigem Kunststoff ausgiesst, nach dem Aushärten des Kunststoffs den oder die Verdrängungskörper reckt und anschliessend aus dem entstandenen Festkörper (44) herauszieht.

15. Verfahren nach Anspruch 14, dadurch gekennzeichnet, dass die langgestreckten Verdrängungskörper monofile Fäden sind.

16. Verfahren nach Anspruch 14 oder 15, dadurch gekennzeichnet, dass man zur Bildung von Verzweigungen zwischen dem Hauptkanal (50) und den Kanälen (46, 48) im Festkörper (44) den den langen und den kurzen Kanal (46, 48) bildenden Faden durch den den Hauptkanal (50) bildenden Faden hindurchzieht und das erhaltene Gebilde in der Form anordnet.

17. Messkammer für die Verwendung in der Vorrichtung gemäss Anspruch 1, gekennzeichnet durch einen Festkörper (44) aus einem gegossenen, im wesentlichen lichtdurchlässigen Kunststoff mit einem Hauptkanal (50), von dem jeweils gewinkelt ein kurzer kapillarer Kanal (46) und ein langer kapillarer Kanal (48) abzweigen.

**Claims**

1. Apparatus for determining the flow shear stress of suspensions, in particular blood, comprising a means having a network of capillary passages, characterized by at least two capillary passages (46, 48) of which the ratio of length $L_l$ of the long passage (48) and the length $L_k$ of the short passage (46) lies in a range of 3 : 1 to 15 : 1, and which are connected to a means for producing a variable pressure differential via a main passage (50), with the passages (46, 48, 50) being provided within a solid body (44) of casted, substantially translucent plastic, and by an optical detector system (22, 38) being horizontally displaceable and by which the flow of the suspension in the capillary passages (46, 48) is detectable.

2. Apparatus according to claim 1, characterized in that the ratio is about 7 : 1.

3. Apparatus according to claim 1, characterized in that the internal diameter of the passages (46, 48) is about 15–1000 µm.

4. Apparatus according to claim 1, characterized in that the passages (46, 48) are connected in U-form to the main passage (50).

5. Apparatus according to claim 1, characterized in that follow filaments of a hydrophobic material are provided in the solid body (44) which comprise the passages (46, 48).

6. Apparatus according to claim 1, characterized in that the means for producing a variable pressure differential in the passages (46, 48) is a pneumatic or hydrodynamic pressure generator (62).

7. Apparatus according to claim 1, characterized in that the pressure generator (62) may be operated in discrete steps of 1–10 Pa.

8. Apparatus according to claim 1, characterized in that a measuring chamber (34) comprising the passages (46, 48) and the main passage (50) is provided between the detector system (22, 38).

9. Apparatus according to claim 8, characterized in that the detector system (22, 38) comprises a light source (74), a magnifying optical system (76, 78, 80), a diaphragm (82) and at least one light detector (84).

10. Apparatus according to claim 9, characterized in that the detector system (22, 38) comprises light sensors corresponding to a plurality of fixed measuring points.

11. Apparatus according to claim 9, characterized in that the detector system (22, 38) is displaceable above and below the measuring chamber (34) manually or by means of a stepping motor.

12. Apparatus according to claim 9, characterized in that the detector system (22, 38) is connected to at least one automatic evaluation unit.

13. Apparatus according to one of claims 1–12, characterized in that in at least one of the passages (46, 48) electrodes are provided for determining the conductivity of the suspensions disposed in the respective passage.

14. Method of making the means comprising a capillary network for use in the apparatus according to claim 1, characterized in that one or more elongated displacement bodies whose diameter and form correspond to the diameter and form of the passages (46, 48) and of the main passage (50) of the means are disposed in a mould, the mould filled with a castable and curable, substantially transparent plastic, after the curing of the plastic the displacement body or bodies are stretched and thereafter removed from the resulting solid body (44).

15. Method according to claim 14, characterized in that the elongated displacement bodies are monofilament threads.

16. Method according to claim 14 or 15, characterized in that to form branches between the main passage (50) and the passages (46, 48) in the solid body (44) the filament forming the long and the short passage (46, 48) is drawn through the filament forming the main passage (50) and the obtained configuration is disposed in the mould.

17. Measuring chamber for the use in the apparatus according to claim 1, characterized by a solid body (44) of a casted, substantially transparent plastic comprising a main passage (50) from which respectively a short capillary passage (46) and a long capillary passage (48) branch in U-form.

**Revendications**

1. Appareil pour déterminer la contrainte tangentielle d'écoulement de suspensions, en particulier du sang, comportant un dispositif qui présente un réseau de canaux capillaires, caractérisé par au moins deux canaux capillaires (46, 48), dans le cas desquels le rapport de la longueur $L_1$ du canal long (48) et de la longueur $L_k$ du canal court (46) se situe sur une plage allant de 3 : 1 à 15 : 1 et qui sont reliés, par l'intermédiaire d'un canal principal (50), à un dispositif de production d'une chute de pression variable, étant précisé que les canaux (46, 48, 50) sont prévus dans un corps solide (44) en plastique coulé, essentiellement transparent; et par un système de détection optique coulissant (22, 38) avec lequel on peut déterminer l'écoulement de la suspension dans les canaux capillaires (46, 48).

2. Appareil selon la revendication 1, caractérisé en ce que le rapport est d'environ 7 : 1.

3. Appareil selon la revendication 1, caractérisé en ce que le diamètre intérieur des canaux (46, 48) se monte à environ 15–1000 microns.

4. Appareil selon la revendication 1, caractérisé en ce que les canaux (46, 48) sont reliés au canal principal (50) sous un certain angle.

5. Appareil selon la revendication 1, caractérisé en ce que dans le corps solide sont prévues des fibres creuses, en matériau hydrophobes, qui représentent les canaux (46, 48, 50).

6. Appareil selon la revendication 1, caractérisé en ce que le dispositif de production d'une chute de pression variable dans les canaux (46, 48) est un générateur de pression pneumatique ou hydrodynamique (62).

7. Appareil selon la revendication 1, caractérisé en ce que le générateur de pression (62) est réglable, par pas discrets, de 1 à 10 Pa.

8. Appareil selon la revendication 1, caractérisé en ce qu'à l'intérieur du système de détection (22, 38) est prévue une chambre de mesure (34) qui présente les canaux (46, 48) et le canal principal (50).

9. Appareil selon la revendication 8, caractérisé en ce que le système de détection (22, 38) présente une source lumineuse (74), une optique de grossissement (76, 78, 80), un prédiaphragme (82) et au moins un détecteur de lumière (84).

10. Appareil selon la revendication 9, caractérisé en ce que le système de détection (22, 38) présente plusieurs détecteurs de lumière correspondant à des points de mesure fixes.

11. Appareil selon la revendication 9, caractérisé en ce que le système de détection (22, 38) peut coulisser au-dessus et au-dessous de la chambre de mesure (34), manuellement ou au moyen d'un moteur pas à pas.

12. Appareil selon la revendication 9, caractérisé en ce que le système de détection (22, 38) est relié à au moins une unité de traitement automatique.

13. Appareil selon l'une des revendications 1–12, caractérisé en ce que dans au moins l'un des canaux (46, 48) sont disposées des électrodes pour déterminer la conductibilité des suspensions qui se trouvent dans le canal en question.

14. Procédé pour fabriquer le dispositif présentant un réseau capillaire pour employer l'appareil selon la revendication 1, caractérisé en ce que l'on dispose, reliés dans un moule, un ou plusieurs corps de déplacement, de forme allongée, dont le diamètre et la forme correspondent au diamètre et à la forme des canaux (46, 48) et du canal principal (50) des dispositifs; en ce que l'on coule dans le moule un plastique moulable et durcissable, sensiblement transparent; en ce qu'après durcissement du plastique, on étire le ou les corps de déplacement et en ce qu'ensuite on les tire hors du corps solide apparu (44).

15. Procédé selon la revendication 14, caractérisé en ce que les corps de déplacement de forme allongée sont des fibres monofilament.

16. Procédé selon la revendication 14 ou 15, caractérisé en ce que, pour former des branchements entre le canal principal (50) et les canaux (46, 48) dans le corps solide (44), on tire les fibres formant le canal long et le canal court (46, 48) à travers la fibre formant le canal principal (50) et on dispose dans le moule la structure obtenue.

17. Chambre de mesure à employer dans l'appareil selon la revendication 1, caractérisée par un corps solide (44) en plastique moulé, sensiblement transparent, comportant un canal principal (50) sur lequel se branchent respectivement, sous un certain angle, un canal capillaire court (46) et un canal capillaire long (48).

FIG.1

FIG.2

# FIG.3

# FIG. 4